# EUROPEAN PATENT SPECIFICATION

(11) **EP 1 840 180 B1**
(45) Date of publication and mention of the grant of the patent: **23.02.2011**
(21) Application number: 07005604.9
(22) Date of filing: 19.03.2007
(51) Int. Cl.: C09J 177/00, C09J 193/04, C09J 201/00

(54) **Use of an adhesive composition for alginate impression material**
Verwendung einer Klebstoffzusammensetzung für Alginatabformmassen
Utilisation d'une composition adhésive pour matériau d'impression à base d'alginate

(30) Priority: 29.03.2006 JP 2006090360
(43) Date of publication of application: 03.10.2007
(73) Proprietor: GC Corporation, Tokyo 174-8585 (JP)
(72) Inventor: Kamohara, Hiroshi, Tokyo 174-8585 (JP); Takeo, Makiko, Tokyo 174-8585 (JP); Fukushima, Shouichi, Tokyo 174-8585 (JP)
(74) Representative: Müller-Boré & Partner Patentanwälte

(56) References cited:
- EP-A1- 0 278 888
- EP-A1- 0 433 871
- WO-A-96/06533
- DE-A1- 1 594 117
- GB-A- 2 190 917
- JP-A- 10 175 812
- JP-A- 2000 160 106
- US-A1- 2003 015 690
- US-A1- 2006 004 120

## Description

The present invention relates to the use of an adhesive composition according to claim 1 for an alginate impression material for applying on an impression tray in advance so as to adhere the impression tray to the alginate impression material, when an impression in an oral cavity is taken by the alginate impression material.

In a dental medical treatment, when a dental prosthesis is produced, the state in an oral cavity and the state of a damaged portion are necessarily reproduced. A dental impression material is used for such the reproduction, and an impression material is built on an impression tray, pressed to contact to a desired portion in an oral cavity, and cured. After curing, the impression material is removed together with the impression tray from the oral cavity.

As a kind of the impression material, an alginate impression material, a silicone impression material, a polyether impression material, an agar impression material and the like have been mainly used. Especially, the alginate impression material has been widely used due to having a comparatively low cost. However, when removing from the inside of an oral cavity, the cured impression material may be peeled from an impressiontray. As a result of this, there is a problem that the impression material is deformed so as not to obtain an impression having high accuracy.

The alginate impression material does not originally have adhesive property to an impression tray made of stainless, plastic or the like, which has been widely used in a dental clinical. So, mechanical holding force is used by providing irregularities, holes or the like on the surface of the impression tray.. However, since the impression material cannot be sufficiently integrated with the impression tray by only the mechanical holding force, a specific adhesive is necessarily used in order to obtain an impression having high accuracy.

However, an adhesive being presently used for such the objective does not have adhesive property to both of the impression tray and the alginate impression material, but have adhesive property to only a metal tray (for example, refer to Japanese Patent Applications Laid Open No. 10-175812, and No. 2000-160106). Further, as the alginate impression material, a type for mixing and kneading an alginate impression material powder with water, and a type for mixing and kneading a paste containing alginate and a curing material powder or a curing material paste have been used. Especially, the paste-type alginate impression material does not originally have adhesive property, and is blended with much amounts of an oil component and a surface active agent so as to be pasted. Thus, the oil component and the surface active agent operate as a separation material. As a result, when the impression material is removed from the inside of an oral cavity, there occurs a problem that the impression material is completely peeled from the impression tray.

As described above, the holding force between the alginate impression material and the impression tray is not sufficient, so that when the impression is taken out from the inside of an oral cavity, there occurs a problem that the impression material is peeled off. Since the conventional adhesive has especially low adhesive property to a plastic impression material, an adhesive having adhesive property to all materials for impression trays has been desired. Since the paste-type alginate impression material has especially low holding force, an objective of the present invention is to provide an adhesive being effective to the paste-type alginate impression material.

The earnest work was carried out in order to solve the above-mentioned problems and, as a result of this, present inventors noted and found out the following phenomenon to complete the present invention: When a specified resin is dissolved in an organic solvent, applied to an impression tray and dried, as an adhesive composition for an alginate impression material, an adhesive membrane is formed on the surface of the impression tray. The membrane can be strongly adhered to the impression tray by the adhesiveness, and the membrane has excellent adhesiveness to both of metal and plastic. Further, when the kneaded alginate impression material is built on the adhesive membrane, the adhesive membrane formed by the specified resin is strongly adhered to the alginate impression material during the curing process of the alginate impression material.

In particular, the present invention is the use of an adhesive composition for an alginate impression material, wherein 1-50 weight % of one or more kinds of a compound selected from rosin, ester gum and polyamide resin are dissolved in an organic solvent.

An adhesive composition for an alginate impression material according to the present invention is an excellent composition, which can be strongly adhered to not only a metal impression tray but also a plastic impression tray, and can surely hold even a paste-type alginate impression material on the impression tray, where the paste-type alginate impression material does not conventionally have sufficient holding force to the impression tray.

An adhesive composition for the use of an alginate impression material according to the present invention is produced by dissolving 1-50 weight % of one or more kinds of compounds selected from rosin, ester gum and polyamide resin in an organic solvent. While there are various kinds of rosin depending on a production method, rosin is basically a mixture of various isomers mainly containing abietic acid. In the present invention, rosin mainly containing abietic acid can be used.

As for ester gum, rosin ester can be used and, for example, methyl, ethyl, or glycerol ester of rosin can be used. Further, as for polyamide resin used in the present invention, polyamide resin synthesized by condensation reaction of polymerized fatty acid with diamine and the like can be used.

When one or more kinds of a compound selected from rosin, ester gum and polyamide resin are dissolved in an organic solvent, the compound becomes a liquid having low viscosity enabling it to be applied to an impression tray by a brush or a spray. Further, after the liquid is applied, the solvent is volatilized so as to form a membrane having adhesiveness to an alginate impression material on the surface of the impression tray. Polyamide resin can be combined to be used with one or more kinds of a compound such as rosin, ester gum. The combination of polyamide resin and rosin or polyamide resin and ester gum is preferable. By combining polyamide resin and rosin or polyamide resin and ester gum, not only an alginate impression material obtained by kneading a powder and water but also a paste alginate impression material can have more preferable adhesiveness to an impression tray, where the paste alginate impression material cannot have sufficient adhesiveness by the conventional adhesive.

In the present invention, the content of such the resin is 1 to 50 weight % in an organic solvent. If the content is less than 1 weight %, the impression tray is not sufficiently adhered to the alginate impression material. If the content is more than 50 weight %, the viscosity of the adhesive composition is increased so as to hardly apply the composition to the impression tray, and thus operatability is reduced. Further, volatilization of the solvent is slow so that the adhering to the impression tray is insufficient. More preferable content is 10 to 30 weight %.

As the organic solvent used in the present invention, isopropyl alcohol, ethyl acetate, and toluene can be used. Such the solvents can be independently used. However, the organic solvent obtained by combining isopropyl alcohol and ethyl acetate or isopropyl alcohol and toluene has high volatility and is comparatively safe, so that it is preferable.

Further, of course, a public known additive such as a colorant, perfume or the like can be used to the adhesive composition for an alginate impression material according to the present invention within the range in which the effect of the present invention is not damaged.

### [Example]

The present invention will be concretely described with examples, but the present invention is not restricted to these examples.

### <Example 1>

| | |
|---|---|
| Polyamide resin | 20 (weight %) |
| Rosin | 1 |
| Isopropyl alcohol | 60 |
| Ethyl acetate . | 19 |

The polyamide resin and rosin were dissolved in a mixed liquid of isopropyl alcohol and ethyl acetate, and thereafter, filled in a glass container. Then, an impression tray was prepared. At this time, three types of the trays were used, that is, a metal tray (the product name: Impression Tray (not having holes), produced by GC Corporation), a plastic tray (the product name: Disposable Spacer Trays, produced by GC America Inc.), and a resin for producing an individual tray (the product name: OSTRON II, produced by GC Corporation). The metal tray and the plastic tray were used in the original states. As for a resin for a tray, the individual tray was produced according to an ordinary method using the resin for a tray.

Then, a maxillary impression was taken by: applying adhesive compositions for alginate impression materials of examples and comparative examples onto these 3 types of the impression trays using a brush; drying those; mixing and kneading 40 ml of distilled water with 16.8 g of a powder-like alginate impression material (the product name: AROMA FINE DFIII, produced by GC Corporation) ; building the mixture on the trays applied with the above-described adhesive compositions; and taking the impression. The effects of the adhesive for an alginate impression material as an adhesive were shown by symbols of A, B, and C. In samples shown by C, an alginate impression material was peeled from an impression tray at the time of removing the impression tray from the inside of an oral cavity for taking an impression. In samples shown by B, when an alginate impression material was taken from the impression tray at the time of removing the inside of an oral cavity, the alginate impression material can be easily taken without remaining on the impression tray. In samples shown by A, when an alginate impression material was taken from an impression tray, the alginate impression material had holding force so as.to be remained on the impression tray.

The paste-like alginate impression material contained 9 weight % of potassium alginate, 0.5 weight % of carrageenan, 0.5 weight % of sodium carbonate, and 90 weight % of water as main material pastes, and contained 40 weight % of calcium sulfate, 1.5 weight % of potassium titanate fluoride, 3 weight % of zinc oxide, 3 weight % of trisodium phosphate, 35.5 weight % of quartz, and 17 weight % of polybutene as curing material pastes. In the paste-like alginate impression material, the main material pastes and the curing material pastes was mixed and kneaded at the weight ratio of 1:1, and after kneading those, the similar test to that of the powder-like alginate impression material was carried out. These results were collectively shown in Table 1.

### <Example 2>

| | |
|---|---|
| Polyamide resin | 10 (weight %) |
| Ester gum | 1 |
| Isopropyl alcohol | 89 |

### <Example 3>

| | |
|---|---|
| Polyamide resin | 30 (weight %) |
| Rosin | 5 |
| Isopropyl alcohol | 40 |
| Ethyl acetate | 25 |

### <Example 4>

| | |
|---|---|
| Polyamide resin | 1 (weight %) |
| Ester gum | 0.2 |
| Isopropyl alcohol | 98.8 |

### <Example 5>

| | |
|---|---|
| Polyamide resin | 15 (weight %) |
| Isopropyl alcohol | 85 |

### <Example 6>

| | |
|---|---|
| Polyamide resin | 45 (weight %) |
| Rosin | 5 |
| Isopropyl alcohol | 30 |
| Ethyl acetate | 20 |

### <Example 7>

| | |
|---|---|
| Ester gum | 35 (weight %) |
| Rosin | 15 |
| Isopropyl alcohol | 30 |
| Ethyl acetate | 20 |

### < Comparative example 1>

| | |
|---|---|
| Polyamide resin | 0.5 (weight %) |
| Rosin | 0.1 |
| Isopropyl alcohol | 99.3 |
| Ethyl acetate | 0.1 |

By using the above-described compositions, an adhesive for an alginate impression material was produced by the similar method to that of Example 1. The similar test to that of Example 1 was carried out, and these results were shown in Table 1.

### <Comparative example2>

The test was carried out so as to be Comparative example 2 like Example 1 except an adhesive was not applied onto an impression tray used in the test of Example 1. These results were shown in Table 1.

**[Table 1]**

| Kinds of Tray | Kinds of Impression material | Examples | | | | | | | Comparative Examples | |
|---|---|---|---|---|---|---|---|---|---|---|
| | | 1 | 2 | 3 | 4 | 5 | 6 | 7 | 1 | 2 |
| Metal | Powder state | A | A | A | A | A | A | A | C | C |
| | Paste state | A | A | A | B | B | A | B | C | C |
| Plastic | Powder state | A | A | A | A | A | A | B | C | C |
| | Paste state | A | A | A | B | B | A | B | C | C |
| Resin for producing individual tray | Powder state | A | A | A | A | A | A | B | C | C |
| | Paste state | A | A | A | B | B | A | B | C | C |

Clearly from Table 1, the adhesive for an alginate impression material obtained by dissolving rosin, ester gum and polyamide resin in an organic solvent could sufficiently hold the alginate impression material with respect to not only the metal impression tray but also the plastic impression tray and the resin for producing an individual tray. More particularly, the adhesive for an alginate impression material made by combinedly using polyamide resin and rosin or polyamide resin and ester gum had proper holding force with respect to both of the powder-like alginate impression material and the paste-like alginate impression material. On the other hand, as for Comparative example 1 in which the content of the compounds was out of the range of claims even though containing the specified compounds used in the present invention, and as for Comparative example 2 in which the adhesive composition for an alginate impression material according to the present invention was not applied, the alginate impression material was peeled from the impression tray, when the impression tray was removed from the inside of an oral cavity.

## Claims

1. The use of an adhesive composition for an alginate impression material, for application to an impression tray so as to adhere the alginate impression material, said composition comprising:
(A) 1 - 50 weight % of one or more members selected from the group consisting of rosin, ester gum and polyamide resin, and
(B) an organic solvent, wherein said 1 - 50 weight % of said one or more members are dissolved in said organic solvent.

## Patentansprüche

1. Verwendung einer Haftmittelzusammensetzung für ein Alginat-Abdruckmaterial zur Anwendung auf einer Abdruckschale, um das Alginat-Abdruckmaterial anzuhaften, wobei die Zusammensetzung
(A) 1 bis 50 Gew.-% eines oder mehrerer Mitglieder, ausgewählt aus der Gruppe, bestehend aus Colophonium, Estergummi und Polyamidharz, und
(B) ein organisches Lösungsmittel, wobei die 1 bis 50 Gew.-% des einen oder der mehreren Mitglieder in dem organischen Lösungsmittel gelöst sind,
umfasst.

## Revendications

1. L'utilisation d'une composition adhésive pour un matériau pour empreintes à base d'alginate, pour application sur un porte-empreintes de manière à faire adhérer le matériau pour empreintes à base d'alginate, ladite composition comprenant :
(A) 1 - 50% en poids d'un ou plusieurs éléments sélectionnés parmi le groupe comprenant de la colophane, de la gomme ester et de la résine polyamide, et
(B) un solvant organique, dans lequel lesdits 1 - 50% en poids du(des)dit(s) un ou plusieurs éléments sont dissouts dans ledit solvant organique.
